# EUROPEAN PATENT APPLICATION

(11) **EP 2 192 103 A1**
(43) Date of publication of application: **02.06.2010**
(21) Application number: 09013880.1
(22) Date of filing: 05.11.2009
(51) Int. Cl.: C07C 51/00, C07C 51/44, C07C 53/08

(54) **Process for the conversion of ethanol to acetic acid**

(30) Priority: 27.11.2008 DK 200801673
(71) Applicant: Haldor Topsoe A/S, 2800 Kgs. Lyngby (DK)
(72) Inventor: Voss, Bodil, 2830 Virum (DK); Schiødt, Niels, Christian, 2700 Brønshøj (DK)

(57) **Abstract**

Process for the preparation of acetic acid comprising the steps of:
(h) providing a feed stream of water and ethanol;
(i) adding the feed stream to a recycle stream comprising unconverted ethanol and water;
(j) heating the admixture to a predetermined reaction temperature and passing the thus heated admixture over a catalyst being active in non-oxidative conversion of ethanol to acetic acid to obtain an effluent being rich in acetic acid;
(k) optionally cooling the effluent;
(1) separating the effluent into a stream rich in acetic acid being essentially free of water, a hydrogen containing stream, and a stream with unconverted amounts of ethanol, water and reactive derivates of acetic acid and optionally ethyl acetate;
(m) recycling the stream with unconverted amounts of ethanol and water to step (a);
(n) determining the amount of water in the recycle stream and adjusting the composition of the ethanol and water feed stream in step (a) to a water/ethanol mole ratio of between 0.3/0.7 to 0.6/0.4.

## Description

The present invention relates to an improved process of converting a stream comprising ethanol and water to obtain a product rich in acetic acid. More particularly, the invention concerns the non-oxidative dehydrogenation of ethanol and water to obtain a product stream of acetic acid essentially free of water.

It has been known for several decades how to produce acetic acid from ethanol.

Ethanol can be produced from ethylene by hydrolysis and it may be produced by fermentation of sugars. Traditionally, hydrolysis of ethylene to produce ethanol has been performed to meet the technical use of ethanol, while fermentation of sugar containing matter is an ancient process the product of which is primarily used for household purposes. In the latter process, the ethanol produced is obtained in an aqueous solution in a concentration of 5-15% by weight along with fermentation by products and solids, the so-called broth.

Typically the ethanol is then distilled in two columns to obtain 96% ethanol and may finally be dried in a bed of zeolites to obtain anhydrous ethanol useful as an additive to gasoline.

As part of a new fuel supply development the capacity of bio-ethanol production for the use as gasoline additive has increased tremendously over the past 10 years, especially in Brazil and the US.

Ethanol can be converted by dehydrogenation to acetic acid via the oxidative and the non-oxidative route, viz.:

EtOH + O₂ = HOAc + H₂O (oxidative route) (1)

EtOH + H₂O = HOAc + 2 H₂ (non-oxidative route) (2)

While the oxidative route is exothermic and not limited by equilibrium, the non-oxidative route is endothermic and equilibrium limited and proceeds via the intermediate acetaldehyde (HAc).

EtOH = HAc + H₂ (non-oxidative) (3)

It is known that e.g. copper is an active catalyst for the non-oxidative dehydrogenation of ethanol to acetic acid. Other catalysts like coal are capable to convert ethanol in the non-oxidative route to acetic acid.

Some of the catalysts being active in the non-oxidative route are active also in the esterification of ethanol and acetic acid, whereby ethyl acetate constitutes part of the product composition by the following reaction:

EtOH + HOAc = EtOAc + H₂O (non-oxidative) (4)

Examples of catalysts active in the oxidative conversion of ethanol to acetic acid are vanadium oxide, gold nanoparticles and supported palladium.

Suggestions of processes for the preparation of acetic acid from ethanol are sparse.

GB 287064 discloses an acetic acid process, where an alcohol such as ethyl alcohol is passed upward in a reactor column containing in a first bed with Ag doped Cu catalyst in its reduced state and in its oxidised state at the top of the reactor. The reduced catalyst is active in dehydrogenation of ethanol to acetaldehyde, which is oxidized by contact with the Cu oxide via acetaldehyde to acetic acid being withdrawn from the top of the reactor. Cu oxide is thereby reduced to copper. The Cu catalyst recovered from the bottom of the reactor may be reoxidized and recycled to the top of the reactor. This process employs a moving bed with Cu/CuO as catalyst and an oxygen carrier for the oxidation of ethanol to acetic acid via acetaldehyde.

Kanichiro Inui et al (Effective formation of ethyl acetate from ethanol over Cu-Zn-Zr-Al-O catalyst', Journal of Molecular Catalysis A: Chemical 216 (2004), page 147-156) describes Cu-Zn-Zr-Al-O as catalysts being active in the conversion of ethanol to ethyl acetate and to acetic acid in presence of water by non-oxidative route. It is mentioned that the selectivity to propanone decreases with increasing selectivity to acetic acid. Up to 15 wt% water in the feed is described, which corresponds to 31% on a mole basis. It is proposed that the reaction proceeds via acetaldehyde, hemiacetal and ethyl acetate to acetic acid through a final hydration.

JP 57102835 discloses a non-oxidative process for the production of acetic acid from ethanol in a first ethanol dehydrogenation reaction over a CuO and other oxidic catalysts and a hydrogen separation step. In a subsequent step acetic acid together with water is separated and acetaldehyde is separated from unconverted ethanol. This process may further comprise a second acetaldehyde dehydrogenation step to acetic acid with addition of additional water, wherein the product of this step is recycled to the hydrogen separation step and unconverted ethanol is recycled to the first ethanol dehydrogenation step.

Separation of acetic acid from water is an expensive process step which must be conducted in equipment being constructed of highly corrosion resistant material. A further disadvantage of the above process is addition of water in a second step in order to provide an oxidant for the conversion of acetaldehyde to acetic acid. The combined extraction and addition of water in such a process scheme imposes a severe economic penalty.

The oxidative route does not offer the possibility of recovering essentially water-free acetic acid without the removal of water by-product.

It has now been found that acetic acid being essentially free of water can be produced by the non-oxidative route from an ethanol/water feed by adjusting the water content in the ethanol feed and adding water to the acetic acid preparation process exclusively together with the ethanol feed.

Pursuant to the above finding this invention provides a Process for the preparation of acetic acid comprising the steps of:
(a) providing a feed stream of water and ethanol;
(b) adding the feed stream to a recycle stream comprising unconverted ethanol and water;
(c) heating the admixture to a predetermined reaction temperature and passing the thus heated admixture over a catalyst being active in non-oxidative conversion of ethanol to acetic acid to obtain an effluent being rich in acetic acid;
(d) optionally cooling the effluent;
(e) separating the effluent into a stream rich in acetic acid being essentially free of water, a hydrogen containing stream, and a stream with unconverted amounts of ethanol, water and reactive derivates of acetic acid and optionally ethyl acetate;
(f) recycling the stream with unconverted amounts of ethanol and water to step (a);
(g) determining the amount of water in the recycle stream and adjusting the composition of the ethanol and water feed stream in step (a) to a water/ethanol mole ratio of between 0.3/0.7 to 0.6/0.4

The production of acetic acid from ethanol may involve production of by-products. In case these by-products or derivatives form azeotropes with the product mixture during the separation process, it may be beneficial to remove water along with the by-product. This is not considered extraction of water but parasitic loss of water.

The separation unit may be a conventional distillation column, where the hydrogen rich stream is retrieved from the gas phase in the reflux drum, the optional acetaldehyde containing stream is withdrawn from the top section of the distillation column and the stream containing the unconverted ethanol is withdrawn from an intermediate section of the column. The acetic acid rich stream is collected from the bottom of the distillation column.

In the process, n-butanol may be formed as a by-product. N-butanol is difficult to separate from acetic acid. By arranging a catalyst being active in the reaction of butanol with acetic acid to butyl acetate in the separation step, it is possible to convert n-butanol to n-butyl acetate and at the same time removing product water generated by the esterification of these.

Zeolites are active in the conversion of n-butanol and acetic acid to n-butyl acetate, thus a bed of zeolite catalyst may advantageously be installed in the distillation column. N-butyl acetate may in turn be removed from the acetic acid product by other means if desired.

If the separation step is performed in the distillation column at a pressure slightly below the pressure prevailing in the reaction step, temperatures from 118 up to about 150°C will be found in the bottom part of the distillation column corresponding to a temperature close to the boiling point of pure acetic acid.

The content of water in the recycle stream of the inventive process depends on the degree of conversion of the intermediate acetaldehyde (reaction 3) to acetic acid, the potential further conversion of the acetic acid with unconverted ethanol to ethyl acetate (reaction 4), the parasitic loss of water in a by-product removal and generated water from by-product or derivatives reactions.

In order to obtain a stable process producing acetic acid essentially free of water from an ethanol and water feed stream, water should only be fed in amounts that secures its complete consumption by the process.

An advantage of the present process is that the separation step results in an acetic acid product being essentially free of water during periods, where in principle the water content of the ethanol/water feed is too high as compared to above criterion. If the separation takes place in a distillation column an increase of the water content in the system may be counteracted by increasing e.g. the distillation column reflux ratio.

Further typical operation conditions comprise temperatures for the catalytic conversion of ethanol to acetic acid of 250-450°C, preferably about 250-350°C and an operation pressure of 0-10 bar, preferably 0-3 bar.

The adjustment of the amount of water in the ethanol feed stream may be managed by establishing a mass balance of the process giving a start target value of water content in the recycle stream, while increasing the water content in the feed after measuring the decrease of water content in the recycle stream and vice versa.

The water content in the recycle stream may be determined by e.g. an online dew point transmitter. Other principles of control may be applied as well.

According to the non-oxidative dehydrogenation reaction 2), equimolar amounts of ethanol and water are required at start conditions of the process.

An equimolar feed of ethanol and water corresponds to a 71.9 wt% ethanol and 28.1 wt% water mixture.

The equimolar feed may be obtained in a side stream from an ethanol plant producing bio-ethanol or fuel-ethanol, de-bottlenecking the process. As an example, such a side stream may be obtained as a vapour stream from a distillation section of an ethanol plant, whereby evaporation of the ethanol/water feed is avoided. It may be advantageous therefore to integrate the inventive process into an ethanol plant, where ethanol and water are present in suitable amounts and especially where the feed is vaporised.

The amount of water in the feed can be adjusted by adding small amounts of steam/water to an un-adjusted feed of ethanol being lean in water.

Suitable ethanol conversion catalysts are any catalyst being active in the conversion of ethanol to acetic acid via acetaldehyde dehydrogenation at the above conditions. Preferred catalysts include those which further catalyse the reaction of ethanol with acetic acid to obtain ethyl acetate. Examples of catalysts active in the dehydrogenation of ethanol to acetic acid are copper based, optionally in combination with zinc oxide, chromium oxide, manganese oxide, zirconium oxide and/or aluminium oxide or a catalyst comprising the above oxides supported on an inert carrier.

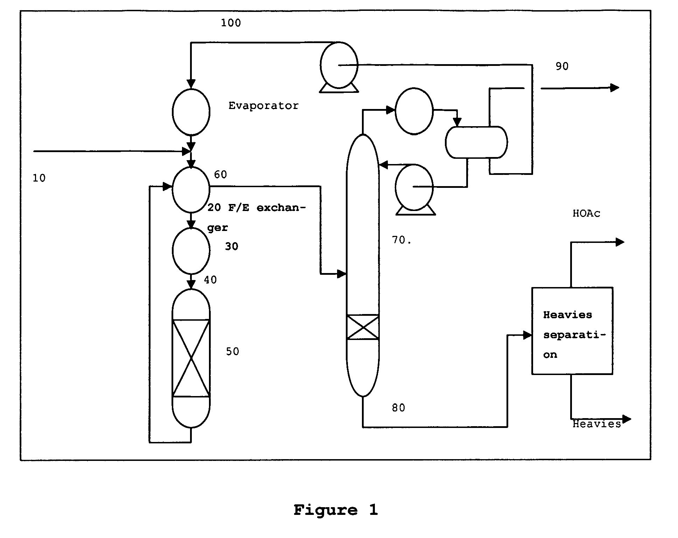

Figure 1 is a simplified flow sheet of an embodiment of the invention, where a stream of evaporated ethanol and water is used as fed to the process. The feed stream is mixed with an evaporated recycle stream primarily comprising unconverted ethanol, acetaldehyde, water and ethyl acetate.

In the following Example condensation products are higher boiling alcohols, subject to esterification in the distillation column provided by the active catalyst bed (e.g. HZSM-5) arranged within a distillation column.

Example 1, below, demonstrates that an adjusted feed stream of ethanol and water results in no extra additions or extractions of water for the production of acetic acid essentially free of water.

### Example 1

Reference is made to Figure 1. A feed 10(1 kmol/h as per example) consisting of adjusted amounts of ethanol and water is added to an evaporated recycle stream 100. The admixture is preheated in feed-effluent heat exchanger 20 and further in preheater 30 in order to reach a proper reaction temperature of the reactor feed 40. The conversion of reactor feed 40 is carried out in acetic acid reactor 50 in presence of a copper aluminate catalyst. The reactor effluent 60 leaving at 320°C is cooled in the F/E exchanger 20 before being passed to distillation column 70. In the distillation column acetic acid product and higher alcohols (heavies) are withdrawn from the bottom of the column in line 80 while hydrogen rich co-product 90 is withdrawn from the overhead of the column. An intermediate boiling fraction comprising acetaldehyde, ethyl acetate, ethanol, water which forms recycle stream 100 is withdrawn as a liquid either from the column overhead together with hydrogen and separated from hydrogen in separator 85. Recycle stream 100 may preferable be withdrawn from column 70 at a lower tray, as shown by the dotted line.

Table 1 summarizes the numbers of a mass balance in the above process being operated with an adjusted ethanol/water feed.

**Table 1**

| Stream no. | 10 | 40 | 60 | 80 | 90 | 100 |
|---|---|---|---|---|---|---|
| Weight % | | | | | | |
| Water | 26.0 | 28.0 | 20.4 | | | 28.8 |
| Ethanol | 74.0 | 30.3 | 9.1 | | | 12.7 |
| Acetaldehyde | | 38.4 | 38.8 | | 18.0 | 53.9 |
| Ethyl acetate | | 3.2 | 3.2 | | | 4.5 |
| Acetic acid | | | 26.1 | 95.9 | | |
| Hydrogen | | | 1.8 | | 82.0 | |
| Condensation | | | 0.7 | 4.1 | | |
| products | | | | | | |
| Flow rate | 32.8 | 119.0 | 114.0 | 30.6 | 2.5 | 81.2 |
| Kg/h | | | | | | |

## Claims

1. Process for the preparation of acetic acid comprising the steps of:
(a) providing a feed stream of water and ethanol;
(b) adding the feed stream to a recycle stream comprising unconverted ethanol and water;
(c) heating the admixture to a predetermined reaction temperature and passing the thus heated admixture over a catalyst being active in non-oxidative conversion of ethanol to acetic acid to obtain an effluent being rich in acetic acid;
(d) optionally cooling the effluent;
(e) separating the effluent into a stream rich in acetic acid being essentially free of water, a hydrogen containing stream, and a stream with unconverted amounts of ethanol, water and reactive derivates of acetic acid and optionally ethyl acetate;
(f) recycling the stream with unconverted amounts of ethanol and water to step (a);
(g) determining the amount of water in the recycle stream and adjusting the composition of the ethanol and water feed stream in step (a) to a water/ethanol mole ratio of between 0.3/0.7 to 0.6/0.4

2. Process of claim 1, wherein the effluent of step (c) further contains acetaldehyde.

3. Process of claim 1, wherein the predetermined reaction conditions in step (c) comprise an operation temperatures for the catalytic conversion of ethanol to ace tic acid of 250-450°C, preferably about 250-350°C and an operation pressure of 0-10 bar, preferably 0-3 bar.

4. Process of claim 1, wherein the acetic acid rich stream is separated from the effluent in step (e) by means of distillation in a distillation column being provided with a bed of a zeolitic catalyst.

5. Process in accordance with anyone of the preceding claims, wherein the feed stream of water and ethanol is a side-stream from an ethanol-plant.

6. Process of claim 5, wherein the side-stream is with drawn from a distillation section of the ethanol-plant.
